(19) Europäisches Patentamt
European Patent Office
Office européen des brevets

(11) EP 0 666 260 B1

(12) **FASCICULE DE BREVET EUROPEEN**

(45) Date de publication et mention de la délivrance du brevet: **04.08.1999 Bulletin 1999/31**

(21) Numéro de dépôt: **95400215.0**

(22) Date de dépôt: **02.02.1995**

(51) Int. Cl.$^6$: **C07D 265/36**, C07D 471/04, C07D 495/04, C07D 413/06, A61K 31/535 // (C07D471/04, 221:00, 209:00), (C07D495/04, 333:00, 221:00)

(54) **Dérivés de 3-(2-aminoéthyl)-4-(3-(trifluorométhyl)benzoyl)-3,4-dihydro-2H-1,4-benzoxazine, leur préparation et leur application en thérapeutique**

3-(2-Aminoethyl)-4-3-(trifluoromethyl)benzoyl-3,4-dihydro-2H-1,4-benzoxazinderivate, deren Herstellung und Verwendung in Therapie

3-(2-aminoethyl)-4-3-(trifluoromethyl)benzoyl-3,4-dihydro-2H-1,4-benzoxazine derivatives, their preparation and use in therapy

(84) Etats contractants désignés:
**AT BE CH DE DK ES FR GB GR IE IT LI LU MC NL PT SE**
Etats d'extension désignés:
**SI**

(30) Priorité: **03.02.1994 FR 9401196**
**03.02.1994 FR 9401197**

(43) Date de publication de la demande:
**09.08.1995 Bulletin 1995/32**

(73) Titulaire: **SYNTHELABO**
**92350 Le Plessis Robinson (FR)**

(72) Inventeurs:
- **George, Pascal**
  **F-78730 St Arnoult en Yvelines (FR)**
- **Frost, Jonathan**
  **F-91320 Wissous (FR)**
- **Pasau, Patrick**
  **F-92220 Bagneux (FR)**
- **Rousselle, Corinne**
  **F-92260 Fontenay Aux Roses (FR)**
- **Bartsch, Régine**
  **F-92260 Fontenay Aux Roses (FR)**
- **Williams, Paul Howard**
  **F-75011 Paris (FR)**
- **Muller, Jean Claude**
  **F-91390 Morsang sur Orge (FR)**

(74) Mandataire: **Ludwig, Jacques et al**
**Sanofi-Synthélabo**
**Service Brevets**
**174, avenue de France**
**75013 Paris (FR)**

(56) Documents cités:
**EP-A- 0 223 344**
**EP-A- 0 302 788**
**EP-A- 0 614 893**
**DE-B- 1 287 584**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7/3.6

## Description

DE-B-12 87 584 divulgue des dérivés de 1,4-benzoxazine ayant une activité analgésique. EP-A-0 614 893 publié le 14.09.94 divulgue certains dérivés de 1,4-benzoxazine qui ont des propriétés neuroprotectrices et antiischémique.

La présente invention a pour objet des dérivés de 3-(2-aminoéthyl)-4-[3-(trifluorométhyl)benzoyl]-3,4-dihydro-2*H*-1,4-benzoxazine, leur préparation et leur application en thérapeutique.

Les composés de l'invention répondent à la formule générale (I)

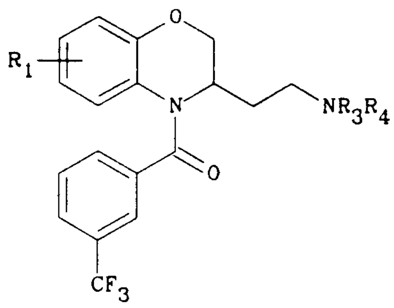

(I)

dans laquelle

$R_1$ représente un atome d'hydrogène, de fluor ou de chlore ou un groupe méthyle, alcoxy en $C_1$-$C_3$ ou nitro,
$R_3$, pris isolément, représente un atome d'hydrogène ou un groupe alkyle en $C_1$-$C_3$,
$R_4$, pris isolément, représente un groupe 2,3-dihydro-1*H*-indén-2-yle, un groupe 2,3-dihydro-1*H*-indén-1-yle ou un groupe 1,2,3,4-tétrahydronaphtalén-1-yle,
ou bien
$R_3$ et $R_4$ forment ensemble, et avec l'atome d'azote qui les porte, un groupe 1,2,3,4-tétrahydro-9*H*-pyrido[3,4-*b*]indol-2-yle, un groupe 1,2,3,4-tétrahydro-9*H*-pyrido[4,3-*b*]indol-3-yle, un groupe 4,5,6,7-tétrahydrothiéno[2,3-*c*]pyridin-6-yle, un groupe 4,5,6,7-tétrahydrothiéno[3,2-*c*]pyridin-6-yle ou un groupe 2,3-dihydro-1*H*-isoindol-2-yle, dont les formules respectives sont les suivantes :

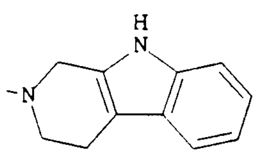 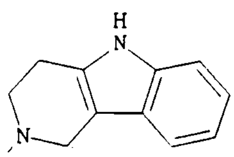 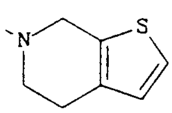 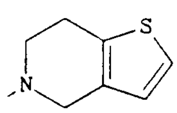 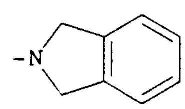

Les composés préférés sont ceux dans la formule desquels $R_1$ représente un atome de fluor ou de chlore ou un groupe méthyle ou méthoxy, $R_3$ représente un atome d'hydrogène ou un groupe alkyle en $C_1$-$C_3$ et $R_4$ représente un groupe 2,3-dihydro-1*H*-indén-2-yle, un groupe 2,3-dihydro-1*H*-indén-1-yle ou un groupe 1,2,3,4-tétrahydronaphtalén-1-yle.

La molécule représentée par la formule générale (I) possédant un atome de carbone asymétrique (position 3 du cycle de benzoxazine), les composés de l'invention peuvent exister sous forme d'énantiomères purs ou de mélange d'énantiomères. De plus, lorsque $R_4$ représente un groupe 2,3-dihydro-1*H*-indén-1-yle ou un groupe 1,2,3,4-tétrahydronaphtalén-1-yle, la molécule comporte un second centre asymétrique. Un composé selon l'invention peut donc exister sous forme d'isomère optique pur ou de mélange de tels isomères.

Enfin, les composés de l'invention peuvent se présenter à l'état de bases libres ou de sels d'addition à des acides.

Conformément à l'invention, on peut préparer les composés de formule générale (I) par un procédé illustré par le schéma de la page suivante.

On fait réagir un 2-aminophénol de formule générale (II), dans laquelle $R_1$ est tel que défini ci-dessus, avec de l'anhydride trifluoracétique, de formule (III), en présence d'une base telle que la pyridine, dans un solvant tel que l'éther.

On obtient un amide de formule générale (IV) que l'on fait réagir avec du 4-bromobut-2-énoate d'éthyle, de

formule (V), en présence d'une base telle que l'éthanolate de sodium, dans un solvant tel que l'éthanol, à une température de l'ordre de 80°C. On réduit ensuite la fonction ester du dérivé de 3,4-dihydro-2*H*-1,4-benzoxazine-3-acétate d'éthyle, de formule générale (VI), soit - dans le cas général - avec un agent réducteur tel que l'hydrure de lithium et d'aluminium, soit - lorsque $R_1$ représente un groupe nitro - avec le borohydrure de sodium dans le 1,1-diméthyléthanol, selon *Synth. Commun.* (1982) **12**(6) 463, pour obtenir le dérivé de 3,4-dihydro-2*H*-1,4-benzoxazine-3-éthanol de formule générale

Schéma

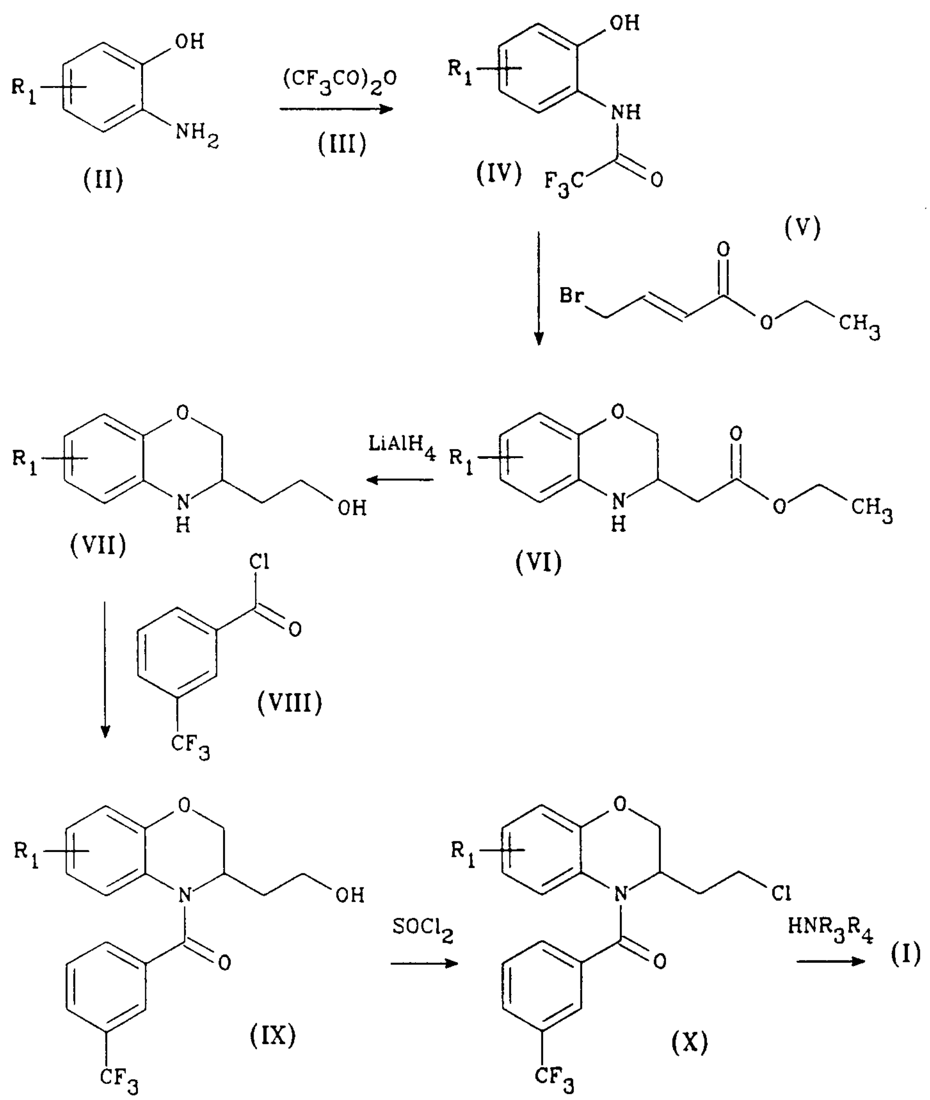


(VII), que l'on fait réagir, dans un solvant tel que le dichlorométhane, avec le chlorure d'acide 3-(trifluorométhyl)benzoïque, de formule (VIII), pour obtenir un alcool de formule générale (IX), que l'on fait réagir avec du chlorure de thionyle pour obtenir un composé de formule générale (X). Finalement, on fait réagir ce dernier avec une amine de formule générale $HNR_3R_4$ dans laquelle $R_3$ et $R_4$ sont tels que définis ci-dessus.

Les produits de départ sont disponibles dans le commerce ou décrits dans la littérature, ou peuvent être synthétisés selon des méthodes qui y sont décrites ou qui sont connues de l'homme du métier.

En particulier, l'aminophénol de formule générale (II) dans laquelle $R_1$ représente un groupe méthoxy est décrit dans *J. Amer. Chem. Soc.* (1949) **71** 1265.

Les amines de formule générale $HNR_3R_4$ dans laquelle $R_4$ représente un groupe 2,3-dihydro-1*H*-indén-2-yle sont décrites dans *J. Med. Chem.* (1980) **23** 745.

Les amines de formule générale $HNR_3R_4$ dans laquelle $R_4$ représente un groupe 2,3-dihydro-1*H*-indén-1-yle sont décrites dans *J. Amer. Chem. Soc.* (1966) **88** 2233.

Les amines de formule générale $HNR_3R_4$ dans laquelle $R_4$ représente un groupe 1,2,3,4-tétrahydronaphtalén-1-yle sont décrites dans *J. Amer. Chem. Soc.* (1960) **82** 459, *C. R. Hebd. Séances Acad. Sci. Ser. C.* (1969) **268** 2225 et *J. Med. Chem.* (1966) **9** 830.

Le 1,2,3,4-tétrahydro-9*H*-pyrido[3,4-*b*]indole est décrit dans *Organic Synthesis* (1971) **51** 136.

Le 1,2,3,4-tétrahydro-9*H*-pyrido[4,3-*b*]indole est décrit dans *J. Chem. Soc. (C)* (1968) 1235.

La 4,5,6,7-tétrahydrothiéno[2,3-*c*]pyridine et la 4,5,6,7-tétrahydrothiéno[3,2-*c*]pyridine sont décrites dans *Arkiv. Kemi* (1970) **13**(19) 217.

La 4,5,6,7-tétrahydrothiéno[3,2-*c*]pyridine est également décrite dans la demande de brevet EP-0342118.

Le 2,3-dihydro-1*H*-isoindole est décrit dans *Organic Synthesis Coll.* (1973) **5** 406 et 1064.

Si on désire obtenir un composé de formule générale (I) optiquement pur, on peut utiliser un alcool de formule générale (IX) optiquement pur, que l'on aura préparé, par exemple, par une méthode enzymatique.

Le principe de base de cette méthode enzymatique consiste à séparer un alcool optiquement pur et l'acétate correspondant, de configuration opposée, par exemple par chromatographie sur colonne de gel de silice.

Selon une première variante, on soumet l'alcool de formule (IX) racémique à une acylation chimique, par exemple au moyen d'anhydride acétique, on hydrolyse de manière stéréospécifique, en présence d'une enzyme, l'un des deux énantiomères de l'acétate racémique, et on sépare l'acétate qui n'a pas été hydrolysé. On obtient un alcool optiquement pur et un acétate optiquement pur de configuration opposée, qui peut, si on le désire, être lui-même hydrolysé par voie chimique ou enzymatique pour fournir le second énantiomère de l'alcool.

Selon une seconde variante on soumet l'alcool de formule (IX) racémique à une acylation stéréospécifique en présence d'une enzyme qui catalyse l'estérification d'un seul des énantiomères, par exemple au moyen d'acétate de vinyle. Comme précédemment, on obtient un alcool optiquement pur et un acétate optiquement pur de configuration opposée, qui peut, si on le désire, être lui même hydrolysé par voie chimique ou enzymatique, pour fournir le second énantiomère de l'alcool.

Dans les deux variantes on peut, selon l'enzyme utilisée, obtenir l'énantiomère lévogyre ou dextrogyre de l'alcool (IX) et son acétate de configuration opposée.

Les enzymes utilisables sont par exemple les lipases de *Mucor Miehei*, de *Penicillium cyclopium* ou de germe de blé.

Par ailleurs, lorsque $R_4$ représente un groupe 2,3-dihydro-1*H*-indén-1-yle ou un groupe 1,2,3,4-tétrahydronaphtalén-1-yle, les composés contiennent, dans leur molécule, un second centre d'asymétrie et existent donc sous forme de diastéréoisomères. L'utilisation d'amines chirales (*J. Amer. Chem. Soc.* (1966) **88** 2233 et *C. R. Hebd. Séances Acad. Sci. Ser. C.* (1969) **268** 2225) permet la synthèse des composés optiquement purs.

Les exemples qui vont suivre illustrent en détail la préparation de quelques composés selon l'invention.

Les microanalyses élémentaires, et les spectres I.R. et R.M.N. confirment les structures des composés obtenus. Les numéros indiqués entre parenthèses dans les titres des exemples correspondent à ceux du tableau donné plus loin.

Exemple 1 (Composé N°1)

(*E*)-2-butènedioate (1:1) de (±)-3-[2-(1,2,3,4-tétrahydro-9*H*-pyrido[3,4-*b*]indol-2-yl)éthyl]-4-[(3-trifluorométhyl)benzoyl]-3,4-dihydro-2*H*-1,4-benzoxazine.

1.1. *N*-(2-hydroxyphényl)trifluoroacétamide.

Dans un réacteur de 4 l, sous agitation magnétique, on prépare une suspension de 1,5 l d'éther diéthylique et 104 g (0,95 mole) de 2-aminophénol et on ajoute 77 ml de pyridine. On refroidit le milieu réactionnel par un mélange de glace et d'éthanol, on ajoute, goutte à goutte, en 1 h, 200 g (0,95 mole) d'anhydride trifluoroacétique, on laisse revenir à température ambiante et on maintient l'agitation pendant 1 h.

On ajoute de l'eau glacée, on sépare la phase organique, on la lave successivement avec de l'acide chlorhydrique 1N, avec de l'eau, avec une solution saturée d'hydrogénocarbonate de sodium et avec une solution saturée de chlorure de sodium. On la sèche sur sulfate de magnésium et on évapore le solvant.

On obtient 170 g de produit qu'on utilise tel quel dans l'étape suivante.

1.2. (±)-3,4-Dihydro-2*H*-1,4-benzoxazine-3-acétate d'éthyle.

Dans un réacteur de 3 l, sous agitation magnétique, refroidi à 0°C, on introduit 650 ml d'éthanol, on ajoute lentement, par petites portions, 5,9 g (0,259 mole) de sodium, puis on ajoute, goutte à goutte, successivement 53 g (0,259

mole) de *N*-(2-hydroxyphényl)trifluoroacétamide et 50 g (0,259 mole) de 4-bromobut-2-énoate d'éthyle pur à 75%, et on chauffe le mélange à 80°C pendant 1,5 h.

On évapore le solvant, on reprend le résidu avec 160 ml d'eau et 65 ml de soude 1N, et on l'extrait par de l'éther diéthylique. On lave la phase organique avec une solution saturée de chlorure de sodium, on la sèche sur sulfate de magnésium et on évapore le solvant. On obtient 38,22 g de produit qu'on purifie par chromatographie sur colonne de gel de silice en éluant avec un mélange de cyclohexane et d'éther isopropylique (1/1).

On obtient 28,02 g de produit.

1.3. (±)-3,4-Dihydro-2*H*-1,4-benzoxazine-3-éthanol.

Dans un réacteur de 1 l, on introduit 190 ml de tétrahydrofurane, on le refroidit avec un mélange de glace et de sel et, sous atmosphère d'argon, on ajoute 7,7 g (0,202 mole) d'hydrure de lithium et d'aluminium puis, goutte à goutte, 28,02 g (0,127 mole) de (±)-3,4-dihydro-2*H*-1,4-benzoxazine-3-acétate d'éthyle en solution dans 190 ml de tétrahydrofurane, et on agite le mélange pendant 3 h.

On refroidit le réacteur avec un mélange de neige carbonique et d'acétone, on ajoute, goutte à goutte, 60 ml d'eau et 30 ml de soude 1N, et on maintient l'agitation pendant 0,5 h.

On élimine le précipité par filtration sur kieselguhr, on le rince successivement avec du tétrahydrofurane et de l'acétate d'éthyle, on évapore les solvants du filtrat, et on obtient 24,99 g de produit brut qu'on purifie par chromatographie sur colonne de gel de silice en éluant avec un mélange de cyclohexane et d'acétate d'éthyle (1/1).

On obtient 15,5 g de produit.

1.4. (±)-4-[3-(Trifluorométhyl)benzoyl]-3,4-dihydro-2*H*-1,4-benzoxazine-3-éthanol.

Dans un ballon de 500 ml on introduit 77 ml de dichlorométhane, 15,93 g (0,072 mole) de (±)-3,4-dihydro-2*H*-1,4-benzoxazine-3-éthanol et 10,67 g (0,077 mole) de carbonate de potassium, on ajoute, goutte à goutte, 16,05 g (0,077 mole) de chlorure de 3-(trifluorométhyl)benzoyle en solution dans 77 ml de dichlorométhane, et on agite le mélange à température ambiante pendant 3,5 h.

On ajoute 72 ml de soude 1N, on sépare la phase organique, on la lave à l'eau puis avec une solution saturée de chlorure de sodium, on la sèche sur sulfate de magnésium et on évapore le solvant. On obtient 33,48 g de produit huileux qu'on purifie par chromatographie sur colonne de gel de silice en éluant avec un mélange de cyclohexane et d'acétate d'éthyle (1/1).

On obtient 21,93 g de produit.

1.5. (±)-3-(2-Chloroéthyl)-4-[3-(trifluorométhyl)benzoyl]-3,4-dihydro-2*H*-1,4-benzoxazine.

A 21,93 g de (±)-4-[3-(trifluorométhyl)benzoyl]-3,4-dihydro-2*H*-1,4-benzoxazine-3-éthanol en solution dans 280 ml de dichlorométhane on ajoute 18 ml (0,248 mole) de chlorure de thionyle, et on agite le mélange à température ambiante pendant 6 h.

On évapore le solvant, on reprend le résidu avec du toluène, on l'évapore, et on purifie l'huile obtenue par chromatographie sur colonne de gel de silice en éluant avec un mélange de cyclohexane et d'éther isopropylique (1/1).

On obtient 21,74 g de produit.

1.6. (*E*)-2-butènedioate (1:1) de (±)-3-[2-(1,2,3,4-tétrahydro-9*H*-pyrido[3,4-*b*]indol-2-yl)éthyl]-4-[(3-trifluorométhyl)benzoyl]-3,4-dihydro-2*H*-1,4-benzoxazine.

A une solution de 2,5 g (0,007 mole) de (±)-3-(2-chloroéthyl)-4-[3-(trifluorométhyl)benzoyl]-3,4-dihydro-2*H*-1,4-benzoxazine dans 20 ml de *N,N*-diméthylformamide, à température ambiante, sous atmosphère d'argon et sous agitation magnétique, on ajoute 1,20 g (0,007 mole) de 1,2,3,4-tétrahydro-9*H*-pyrido[3,4-*b*]indole, 1,45 g (0,0105 mole) de carbonate de potassium et 1,16 g (0,007 mole) d'iodure de potassium, et on chauffe le mélange à 160°C pendant 1 h.

On le refroidit, on ajoute 80 ml d'eau et 80 ml d'acétate d'éthyle, on sépare les phases, on extrait la phase aqueuse avec deux fois 80 ml d'acétate d'éthyle, on rassemble les phases organiques, on les lave avec 100 ml de solution saturée de chlorure de sodium, on les sèche sur sulfate de magnésium, on filtre et on évapore le solvant. On obtient 4 g de produit huileux qu'on purifie par chromatographie sur colonne de gel de silice en éluant avec un mélange de cyclohexane et d'acétate d'éthyle (7/3).

On obtient 3,5 g de base pure sous forme d'huile jaune. On prépare le fumarate en ajoutant un équivalent d'acide fumarique, on l'isole et on le recristallise, sous forme de cristaux blancs, dans l'éthanol.

On isole finalement 1,71 g de fumarate.

Point de fusion : 211-212°C.

Exemple 2 (Composé N°12)

(*E*)-2-butènedioate (1:1) de (±)-6-fluoro-3-[2-(4,5,6,7-tétrahydrothiéno[2,3-*c*]pyridin-6-yl)éthyl]-4-[(3-trifluorométhyl)benzoyl]-3,4-dihydro-2*H*-1,4-benzoxazine.

2.1. 2-Amino-4-fluorophénol.

A une solution de 50 g (0,318 mole) de 4-fluoro-2-nitrophénol dans 1,5 l d'eau, sous agitation magnétique, on ajoute 120 g (0,689 mole) d'hydrosulfite de sodium, on chauffe le mélange au reflux, on rajoute 120 g (0,689 mole) d'hydrosulfite de sodium et on poursuit le chauffage au reflux pendant 0,75 h.

On refroidit le mélange, on ajoute, par petites portions, de l'hydrogénocarbonate de sodium jusqu'à pH basique, on ajoute 1 l d'éther diéthylique, on sépare les phases, on extrait la phase aqueuse avec 1 l d'éther diéthylique, on rassemble les phases organiques, on les lave avec 1 l d'eau et 1 l de solution saturée de chlorure de sodium, on les sèche sur sulfate de magnésium, on filtre et on évapore le solvant.

On obtient 21,1 g de produit qu'on utilise tel quel dans l'étape suivante.

2.2. *N*-(5-fluoro-2-hydroxyphényl)trifluoroacétamide.

Dans un réacteur de 1 l, sous agitation magnétique, on prépare une suspension de 260 ml d'éther diéthylique et 21 g (0,165 mole) de 2-amino-4-fluorophénol, on ajoute 14 ml de pyridine, on refroidit le milieu réactionnel avec un mélange de glace et d'éthanol, on ajoute, goutte à goutte, en 1 h, 23,3 ml (0,165 mole) d'anhydride trifluoroacétique, on laisse revenir à température ambiante et on maintient l'agitation pendant 3 h.

On ajoute de l'eau glacée, on sépare la phase organique, on la lave successivement avec 170 ml d'acide chlorhydrique 1N, avec de l'eau, avec une solution saturée d'hydrogénocarbonate de sodium et avec une solution saturée de chlorure de sodium. On la sèche sur sulfate de magnésium et on évapore le solvant.

On obtient 32,7 g de produit qu'on utilise tel quel dans l'étape suivante.

2.3. (±)-6-Fluoro-3,4-dihydro-2*H*-1,4-benzoxazine-3-acétate d'éthyle.

Dans un réacteur de 1 l, sous agitation magnétique, refroidi à 0°C, on introduit 370 ml d'éthanol, on ajoute lentement, par petites portions, 3,5 g (0,146 mole) de sodium, puis on ajoute, goutte à goutte, successivement 32,54 g (0,146 mole) de *N*-(5-fluoro-2-hydroxyphényl)trifluoroacétamide et 28,18 g (0,146 mole) de 4-bromobut-2-énoate d'éthyle pur à 75%, et on chauffe le mélange à 80°C pendant 3 h.

On évapore le solvant, on reprend le résidu avec 90 ml d'eau et 37 ml de soude 1N, et on l'extrait par de l'éther diéthylique. On lave la phase organique avec une solution saturée de chlorure de sodium, on la sèche sur sulfate de magnésium et on évapore le solvant. On obtient 38,74 g de produit qu'on purifie par chromatographie sur colonne de gel de silice en éluant avec un mélange de cyclohexane et d'éther isopropylique (1/1).

On obtient 23,22 g de produit.

2.4. (±)-6-Fluoro-3,4-dihydro-2*H*-1,4-benzoxazine-3-éthanol.

Dans un réacteur de 1 l, on introduit 145 ml de tétrahydrofurane, on le refroidit avec un mélange de glace et de sel et, sous atmosphère d'argon, on ajoute 5,9 g (0,153 mole) d'hydrure de lithium et d'aluminium puis, goutte à goutte, 23,22 g (0,097 mole) de (±)-6-fluoro-3,4-dihydro-2*H*-1,4-benzoxazine-3-acétate d'éthyle en solution dans 145 ml de tétrahydrofurane, et on agite le mélange pendant 2,5 h.

On refroidit le réacteur avec un mélange de neige carbonique et d'acétone, on ajoute, goutte à goutte, 45 ml d'eau et 23 ml de soude 1N, et on maintient l'agitation pendant 0,5 h.

On élimine le précipité par filtration sur kieselguhr, on le rince successivement avec du tétrahydrofurane et de l'acétate d'éthyle, on évapore les solvants du filtrat et on obtient 18,07 g de produit brut qu'on purifie par chromatographie sur colonne de gel de silice en éluant avec un mélange de cyclohexane et d'acétate d'éthyle (1/1).

On obtient 13,9 g de produit.

2.5. (±)-6-Fluoro-4-[3-(trifluorométhyl)benzoyl]-3,4-dihydro-2*H*-1,4-benzoxazine-3-éthanol.

Dans un ballon de 500 ml on introduit 70 ml de dichlorométhane, 13,69 g (0,069 mole) de (±)-6-fluoro-3,4-dihydro-2*H*-1,4-benzoxazine-3-éthanol et 10,27 g (0,074 mole) de carbonate de potassium, on ajoute, goutte à goutte,

15,44 g (0,074 mole) de chlorure de 3-(trifluorométhyl)benzoyle en solution dans 70 ml de dichlorométhane, et on agite le mélange à température ambiante pendant 5 h.

On ajoute 70 ml de soude 1N, on sépare la phase organique, on la lave à l'eau puis avec une solution saturée de chlorure de sodium, on la sèche sur sulfate de magnésium et on évapore le solvant. On obtient 27,53 g de produit huileux qu'on purifie par chromatographie sur colonne de gel de silice en éluant avec un mélange de cyclohexane et d'acétate d'éthyle (2/1).

On obtient 11,27 g de produit.

2.6. (±)-3-(2-Chloroéthyl)-6-fluoro-4-[3-(trifluorométhyl)benzoyl]-3,4-dihydro-2*H*-1,4-benzoxazine.

A 11,27 g (0,031 mole) de (±)-6-fluoro-4-[3-(trifluorométhyl)benzoyl]-3,4-dihydro-2*H*-1,4-benzoxazine-3-éthanol en solution dans 140 ml de dichlorométhane on ajoute 9 ml (0,124 mole) de chlorure de thionyle, et on agite le mélange à température ambiante pendant 5 h.

On évapore le solvant, on reprend le résidu avec du toluène, on l'évapore, et on purifie l'huile obtenue par chromatographie sur colonne de gel de silice en éluant avec un mélange de cyclohexane et d'éther isopropylique (1/1).

On obtient 10.33 g de produit.

2.7. (*E*)-2-butènedioate (1:1) de (±)-6-fluoro-3-[2-(4,5,6,7-tétrahydrothiéno[2,3-*c*]pyridin-6-yl)éthyl]-4-[(3-trifluorométhyl)benzoyl]-3,4-dihydro-2*H*-1,4-benzoxazine.

A une solution de 1,11 g (0,003 mole) de (±)-3-(2-chloroéthyl)-6-fluoro-4-[3-(trifluorométhyl)benzoyl]-3,4-dihydro-2*H*-1,4-benzoxazine dans 15 ml de *N,N*-diméthylformamide, à température ambiante, sous argon et sous agitation magnétique, on ajoute 0,66 g (0,003 mole) d'oxalate de 4,5,6,7-tétrahydrothiéno[2,3-*c*]pyridine, 1,19 g (0,0086 mole) de carbonate de potassium et 0,5 g (0,003 mole) d'iodure de potassium, et on chauffe le mélange à 160°C pendant 1 h.

On le refroidit, on ajoute 50 ml d'eau et 50 ml éther diéthylique, on sépare les phases, on extrait la phase aqueuse avec deux fois 50 ml d'éther diéthylique, on rassemble les phases organiques, on les lave avec 50 ml de solution saturée de chlorure de sodium, on les sèche sur sulfate de magnésium, on filtre et on évapore le solvant. On obtient 1,56 g de produit huileux qu'on purifie par chromatographie sur colonne de gel de silice en éluant avec un mélange de cyclohexane et d'acétate d'éthyle (7/3).

On obtient 0,80 g de base sous forme d'huile jaune.

On prépare le fumarate en ajoutant un équivalent d'acide fumarique, on l'isole et on le recristallise, sous forme de cristaux blancs, dans le propan-2-ol.

On isole finalement 0,77 g de fumarate.

Point de fusion : 181-182°C.

<u>Exemple 3</u> (Composé N°24)

Ethanedioate (1:1) de (±)-3-[2-[(2,3-dihydro-1*H*-indén-2-yl)méthylamino]éthyl]-6-méthoxy-4-[3-(trifluorométhyl)benzoyl]-3,4-dihydro-2*H*-1,4-benzoxazine.

3.1. *N*-(2-hydroxy-5-méthoxyphényl)trifluoroacétamide.

Dans un réacteur de 2 l, sous agitation magnétique, on prépare une suspension de 1 l d'éther diéthylique et 75,33 g (0,54 mole) de 2-amino-4-méthoxyphénol, et on ajoute 56 ml de pyridine. On refroidit le milieu réactionnel par un mélange de glace et d'éthanol, on ajoute, goutte à goutte, en 1 h, 131,4 g (0,625 mole) d'anhydride trifluoroacétique, on laisse revenir à température ambiante et on maintient l'agitation pendant 2 h.

On ajoute de l'eau glacée, on sépare la phase organique, on la lave successivement avec 500 ml d'acide chlorhydrique 1N, avec de l'eau, avec une solution saturée d'hydrogénocarbonate de sodium et avec une solution saturée de chlorure de sodium. On la sèche sur sulfate de magnésium et on évapore le solvant.

On obtient 41,6 g de produit qu'on utilise tel quel dans l'étape suivante.

3.2. (±)-6-Méthoxy-3,4-dihydro-2*H*-1,4-benzoxazine-3-acétate d'éthyle.

Dans un réacteur de 1 l, sous agitation magnétique, refroidi à 0°C, on introduit 450 ml d'éthanol, on ajoute lentement, par petites portions, 7 g (0,32 mole) de sodium, puis on ajoute, goutte à goutte, successivement 37,94 g (0,16 mole) de *N*-(2-hydroxy-5-méthoxyphényl)trifluoroacétamide et 41,2 g (0,16 mole) de 4-bromobut-2-énoate d'éthyle pur

à 75%, et on chauffe le mélange à 80°C pendant 2 h.

On évapore le solvant, on reprend le résidu avec 100 ml d'eau et 40 ml de soude 1N, et on l'extrait par de l'éther diéthylique. On lave la phase organique avec une solution saturée de chlorure de sodium, on la sèche sur sulfate de magnésium et on évapore le solvant. On obtient 38,46 g de produit qu'on purifie par chromatographie sur colonne de gel de silice en éluant avec un mélange de cyclohexane et d'éther isopropylique (1/1).

On obtient 21,22 g de produit.

3.3. (±)-6-Méthoxy-3,4-dihydro-2*H*-1,4-benzoxazine-3-éthanol.

Dans un réacteur de 1 l, on introduit 130 ml de tétrahydrofurane, on le refroidit avec un mélange de glace et de sel et, sous atmosphère d'argon, on ajoute 5 g (0,132 mole) d'hydrure de lithium et d'aluminium puis, goutte à goutte, 20,73 g (0,0825 mole) de (±)-6-méthoxy-3,4-dihydro-2*H*-1,4-benzoxazine-3-acétate d'éthyle en solution dans 130 ml de tétrahydrofurane, et on agite le mélange pendant 1,5 h.

On refroidit le réacteur avec un mélange de neige carbonique et d'acétone, on ajoute, goutte à goutte, 40 ml d'eau et 20 ml de soude 1N, et on maintient l'agitation pendant 0,5 h.

On élimine le précipité par filtration sur kieselguhr, on le rince successivement avec du tétrahydrofurane et de l'acétate d'éthyle, on évapore les solvants du filtrat, et on obtient 25,15 g de produit brut qu'on purifie par chromatographie sur colonne de gel de silice en éluant avec un mélange de cyclohexane et d'acétate d'éthyle (1/1).

On obtient 10,53 g de produit.

3.4. (±)-6-Méthoxy-4-[3-(trifluorométhyl)benzoyl]-3,4-dihydro-2*H*-1,4-benzoxazine-3-éthanol.

Dans un ballon de 500 ml on introduit 80 ml de dichlorométhane, 10,53 g (0,05 mole) de (±)-6-méthoxy-3,4-dihydro-2*H*-1,4-benzoxazine-3-éthanol et 7,4 g (0,0535 mole) de carbonate de potassium, on ajoute, goutte à goutte, 11,16 g (0,0535 mole) de chlorure de 3-(trifluorométhyl)benzoyle en solution dans 80 ml de dichlorométhane, et on agite le mélange à température ambiante pendant 2 h.

On ajoute 50 ml de soude 1N, on sépare la phase organique, on la lave à l'eau puis avec une solution saturée de chlorure de sodium, on la sèche sur sulfate de magnésium et on évapore le solvant. On obtient 22,25 g de produit huileux qu'on purifie par chromatographie sur colonne de gel de silice en éluant avec un mélange de cyclohexane et d'acétate d'éthyle (2/1).

On obtient 14,74 g de produit.

3.5. (±)-3-(2-Chloroéthyl)-6-méthoxy-4-[3-(trifluorométhyl)benzoyl]-3,4-dihydro-2*H*-1,4-benzoxazine.

A 14,74 g (0,039 mole) de (±)-6-méthoxy-4-[3-(trifluorométhyl)benzoyl]-3,4-dihydro-2*H*-1,4-benzoxazine-3-éthanol en solution dans 165 ml de dichlorométhane on ajoute 14 ml (0,19 mole) de chlorure de thionyle, et on agite le mélange à température ambiante pendant 5 h.

On évapore le solvant, on reprend le résidu avec du toluène, on l'évapore à sec, et on purifie l'huile obtenue par chromatographie sur colonne de gel de silice en éluant avec un mélange de cyclohexane et d'éther isopropylique (1/1).

On obtient 14,7 g de produit.

3.6. Ethanedioate (1:1) de (±)-3-[2-[(2,3-dihydro-1*H*-indén-2-yl)méthylamino]éthyl]-6-méthoxy-4-[3-(trifluorométhyl)benzoyl]-3,4-dihydro-2*H*-1,4-benzoxazine.

A une solution de 1,6 g (0,004 mole) de (±)-3-(2-chloroéthyl)-6-méthoxy-4-[3-(trifluorométhyl)benzoyl]-3,4-dihydro-2*H*-1,4-benzoxazine dans 18 ml de *N,N*-diméthylformamide, à température ambiante, sous atmosphère d'argon et sous agitation magnétique, on ajoute 0,73 g (0,004 mole) de chlorhydrate de *N*-méthyl-2,3-dihydro-1*H*-indène-2-amine, 1,38 g (0,01 mole) de carbonate de potassium et 0,66 g (0,004 mole) d'iodure de potassium, et on chauffe le mélange à 150°C pendant 1 h.

On le refroidit, on ajoute 38 ml d'eau et 70 ml d'éther diéthylique, on sépare les phases, on extrait la phase aqueuse avec deux fois 70 ml d'éther diéthylique, on rassemble les phases organiques, on les lave avec 70 ml de solution saturée de chlorure de sodium, on les sèche sur sulfate de magnésium, on filtre et on évapore le solvant. On obtient 1,91 g de produit huileux qu'on purifie par chromatographie sur colonne de gel de silice en éluant avec un mélange de dichlorométhane et de méthanol (85/15).

On obtient 0,940 g de base pure sous forme d'huile jaune.

On prépare l'oxalate en ajoutant un équivalent d'acide oxalique, on l'isole et on le recristallise, sous forme de cristaux blancs, dans l'acétate d'éthyle.

On isole finalement 0,370 g d'oxalate.

Point de fusion : 185-187°C.

Exemple 4 (Composé N°30)

(*E*)-2-butènedioate (1:1) de 6-chloro-3-[2-[(1,2,3,4-tétrahydronaphtalén-1-yl)méthylamino]éthyl]-4-[3-(trifluorométhyl)benzoyl]-3,4-dihydro-2*H*-1,4-benzoxazine, mélange 65:35 de (±)-diastéréoisomères.

4.1. *N*-(5-Chloro-2-hydroxyphényl)trifluoroacétamide.

Dans un réacteur de 1 litre, sous agitation magnétique, on met en suspension, dans 320 ml d'éther diéthylique, 25 g (0,174 mole) de 2-amino-4-chlorophénol, on ajoute 18 ml de pyridine, on refroidit le milieu avec un mélange de glace et d'éthanol, on ajoute, goutte à goutte, en 1 heure, 24,6 ml (0,174 mole) d'anhydride trifluoroacétique, on laisse revenir à température ambiante et on poursuit l'agitation pendant 1 heure.
On ajoute de l'eau glacée, on décante, on lave la phase organique sucessivement avec 320 ml de solution 1 N d'acide chlorhydrique, avec de l'eau, avec une solution saturée d'hydrogénocarbonate de sodium, puis une solution saturée de chlorure de sodium, on la sèche sur sulfate de magnésium, et on évapore le solvant. On obtient 40,3 g de produit qu'on utilise tel quel dans l'étape suivante.

4.2. (±) 6-chloro-3,4-dihydro-2*H*-1,4-benzoxazine-3-acétate d'éthyle.

Dans un réacteur de 3 litres, sous agitation magnétique, refroidi à 0°C, on introduit 420 ml d'éthanol, on ajoute lentement, par petites portions, 3,8 g (0,166 mole) de sodium, puis, successivement, et au goutte à goutte, 40 g (0,166 mole) de *N*-(5-chloro-2-hydroxyphényl)trifluoroacétamide et 40 g (0,155 mole) de 4-bromobut-2-énoate d'éthyle pur à 75%, et on chauffe le mélange à 85°C pendant 2 heures. On évapore le solvant, on reprend le résidu avec 100 ml d'eau et 40 ml de soude 1 N et on l'extrait avec de l'éther diéthylique. On sépare la phase organique, on la lave avec une solution aqueuse saturée de chlorure de sodium, on la sèche sur sulfate de magnésium et on l'évapore. On obtient 28,58 g de produit qu'on purifie par chromatographie sur colonne de gel de silice en éluant avec un mélange cyclohexane/éther isopropylique (1/1).
On obtient 23,72 g de produit.

4.3. (±) 6-Chloro-3,4-dihydro-2*H*-1,4-benzoxazine-3-éthanol.

Dans un réacteur de 1 litre on place 150 ml de tétrahydrofurane, on le refroidit avec un mélange de glace et de sel et, sous atmosphère d'argon, on ajoute 5,92 g (0,156 mole) d'hydrure de lithium et d'aluminium puis, goutte à goutte, 23,52 g (0,0973 mole) de (±) 6-chloro-3,4-dihydro-2*H*-1,4-benzoxazine-3-acétate d'éthyle en solution dans 150 ml de tétrahydrofurane, et on agite le mélange pendant 1,5 heure.
On refroidit le réacteur avec un mélange de neige carbonique et d'acétone, et on ajoute, goutte à goutte, 40 ml d'eau et 20 ml de soude 1 N, on agite pendant 0,5 heure, on filtre le précipité sur kieselguhr, on le rince avec du tétrahydrofurane puis avec de l'acétate d'éthyle, et on évapore le solvant. On isole 27,5 g de produit brut qu'on purifie par chromatographie sur colonne de gel de silice en éluant avec un mélange cyclohexane/acétate d'éthyle (1/1).
On obtient 19,67 g de produit.

4.4. (±) 6-Chloro-4-[(3-trifluorométhyl)benzoyl]-3,4-dihydro-2*H*-1,4-benzoxazine-3-éthanol.

Dans un ballon de 1 litre on introduit 100 ml de dichlorométhane, 19,17 g (0,09 mole) de (±) 6-chloro-3,4-dihydro-2*H*-1,4-benzoxazine-3-éthanol, 13,3 g (0,096 mole) de carbonate de potassium et on ajoute, goutte à goutte, 20 g (0,096 mole) de chlorure de 3-(trifluorométhyl)benzoyle en solution dans 100 ml de dichlorométhane, et on agite le mélange à température ambiante pendant 3 heures.
On ajoute 90 ml de soude 1 N, on sépare la phase organique, on la lave à l'eau puis avec une solution saturée de chlorure de sodium, on la sèche sur sulfate de magnésium et on évapore le solvant. On obtient 36 g de produit huileux qu'on purifie par chromatographie sur colonne de gel de silice en éluant avec un mélange cyclohexane/acétate d'éthyle (2/1).
On obtient 24,21 g de produit.

4.5. (±) 6-Chloro-3-(2-chloroéthyl)-4-[(3-trifluorométhyl)benzoyl]-3,4-dihydro-2*H*-1,4-benzoxazine.

A 24,21 g de (±) 6-chloro-4-[(3-trifluorométhyl)benzoyl]-3,4-dihydro-2*H*-1,4-benzoxazine-3-éthanol en solution dans 260 ml de dichlorométhane on ajoute 18 ml (0,25 mole) de chlorure de thionyle et on agite le mélange à température ambiante pendant 6 heures.
On évapore le solvant, on reprend le résidu avec du toluène et on l'évapore. On purifie les 25,21 g d'huile obtenue par chromatographie sur colonne de gel de silice en éluant avec un mélange cyclohexane/éther isopropylique (1/1).
On obtient 23,73 g de produit.

4.6. (*E*)-2-butènedioate (1:1) de (±)-6-chloro-3-[2-[(1,2,3,4-tétrahydronaphtalén-1-yl)méthylamino]éthyl]-4-[3-(trifluorométhyl)benzoyl]-3,4-dihydro-2*H*-1,4-benzoxazine.

A une solution de 1,8 g (0,0044 mole) de (±)-6-chloro-3-(2-chloroéthyl)-4-[(3-trifluorométhyl)benzoyl]-3,4-dihydro-2*H*-1,4-benzoxazine dans 13 ml de *N,N*-diméthylformamide, à température ambiante, sous atmosphère d'argon et sous agitation magnétique, on ajoute 0,88 g (0,0044 mole) de chlorhydrate de *N*-méthyl-1,2,3,4-tétrahydronaphtalène-1-amine, 1,23 g (0,009 mole) de carbonate de potassium et 0,74 g (0,0044 mole) d'iodure de potassium, et on chauffe le mélange à 120°C pendant 1 h30.
On le refroidit, on ajoute 50 ml d'eau, on obtient un produit collant qu'on lave à l'eau, on le dissout dans 50 ml d'acétate d'éthyle, on sèche la solution sur sulfate de magnésium, on la filtre , et on évapore le solvant sous pression réduite. On obtient 2,39 g de produit huileux qu'on purifie par chromatographie sur colonne de gel de silice en éluant avec un mélange de de cyclohexane et d'acétate d'éthyle (8/2).
On obtient 1,62 (0,003 mole) g de base.
On prépare le fumarate en ajoutant 0,35 g (0,003 mole) d'acide fumarique dans 15 ml de propan-2-ol, on chauffe le mélange au reflux, on évapore le solvant sous pression réduite, on reprend le résidu avec de l'éther diéthylique, on sépare le précipité par filtration et on le recristallise dans le propan-2-ol. Après séchage à chaud en présence de pentoxyde de phosphore on isole finalement 0,3 g de fumarate.

Point de fusion : 145-146°C (mélange 65:35 de (±)-diastéréo-isomères).

<u>Exemple 5</u> (Composé N°38)

(*E*)-2-butènedioate (1:2) de (+)-6-chloro-3-[2-[(2,3-dihydro-1*H*-indén-2-yl)méthylamino]éthyl]-4-[3-(trifluorométhyl)benzoyl]-3,4-dihydro-2*H*-1,4-benzoxazine.

5.1. (-)-6-Chloro-4-[(3-trifluorométhyl)benzoyl]-3,4-dihydro-2*H*-1,4-benzoxazine-3-éthanol.

On dissout 5,7 g (0,0148 mole) de (±) 6-chloro-4-[(3-trifluorométhyl)benzoyl]-3,4-dihydro-2*H*-1,4-benzoxazine-3-éthanol dans 60 ml d'acétate d'éthyle, on ajoute 570 mg de lipase de *Mucor miehei* sur résine à 10% (Lipozyme IM60™, Novo™) et 1,36 ml d'acétate de vinyle, et on agite le mélange à température ambiante en suivant le déroulement de la réaction par HPLC sur colonne chirale, en éluant avec un mélange d'hexane et de propan-2-ol (95/5).
Après 3 jours de réaction le taux de conversion est de 52%, avec un excès énantiomérique ee en alcool supérieur à 98%. On filtre le mélange sur kieselguhr, en rinçant le solide avec 10 ml d'acétate d'éthyle, on concentre le filtrat sous pression réduite, et on sépare le mélange d'ester et d'alcool lévogyre par chromatographie flash en utilisant un mélange d'acétate d'éthyle et de cyclohexane 1/9 pour éluer l'ester et un mélange, puis 4/6 pour éluer l'alcool.
On obtient 2,5 g d'alcool lévogyre et 3,0 g d'ester.

Alcool : $[\alpha]_D^{25}$ = -48° (c=0,5 ; $CHCl_3$). ee=98,3%.
Ester : ee=91%.

5.2. (*E*)-2-butènedioate (1:2) de (+)-6-chloro-3-[2-[(2,3-dihydro-1*H*-indén-2-yl)méthylamino]éthyl]-4-[3-(trifluorométhyl)benzoyl]-3,4-dihydro-2*H*-1,4-benzoxazine.

On opère dans des conditions analogues à celles décrites dans les exemples précédents, en traitant le (-)-6-chloro-4-[(3-trifluorométhyl)benzoyl]-3,4-dihydro-2*H*-1,4-benzoxazine-3-éthanol par le chlorure de thionyle, pour obtenir la 6-chloro-3-(2-chloroéthyl)-4-[(3-trifluorométhyl)benzoyl]-3,4-dihydro-2*H*-1,4-benzoxazine (dont on n'a pas déterminé le pouvoir rotatoire), puis en faisant réagir cette dernière avec la *N*-méthyl-2,3-dihydro-1*H*-indène-2-amine, puis en traitant la base avec de l'acide fumarique.

Point de fusion : 168-169°C $[\alpha]_D^{20}$ = +16,2° (c=1 ; $CH_3OH$).

Exemple 6 (Composé N°39)

(*E*)-2-butènedioate (1:2) de (-)-6-chloro-3-[2-[(2,3-dihydro-1*H*-indén-2-yl)méthylamino]éthyl]-4-[3-(trifluorométhyl)benzoyl]-3,4-dihydro-2*H*-1,4-benzoxazine.

6.1. (+)-6-Chloro-4-[(3-trifluorométhyl)benzoyl]-3,4-dihydro-2*H*-1,4-benzoxazine-3-éthanol.

On dissout les 3,0 g d'ester obtenus selon l'exemple 5.1 dans 14 ml de toluène, on ajoute 70 ml de tampon phosphate 0,3 M à pH=7,3, on agite vigoureusement le mélange à température ambiante jusqu'à formation d'une émulsion, puis on ajoute 300 mg de lipase libre de *Mucor miehei* (Biocatalyst™) et on suit le déroulement de la réaction par HPLC sur colonne chirale, comme décrit dans l'exemple 5.1.
Après 67h de réaction le taux de conversion est de 28% et le pH est de 6,95 ; on l'ajuste à 7,5 avec de la soude 4N, on chauffe le mélange à 35°C et on poursuit l'agitation.
Après 48h de réaction le taux de conversion est de 67% ; on réajuste le pH à 7 5, on ajoute 300 mg d'enzyme et on poursuit l'agitation.
Après 32h de réaction le taux de conversion est de 82% et l'excès énantiomérique de l'alcool est de 98,1%. On dilue alors le mélange avec 100 ml d'éther diéthylique, on la filtre sur kieselguhr, on extrait la phase aqueuse quatre fois avec 100 ml d'éther diéthylique, on sèche la phase organique sur sulfate de magnésium, on évapore le solvant sous pression réduite et on purifie le résidu par chromatographie, comme décrit dans l'exemple 5.1.
On obtient 2,15 g d'alcool dextrogyre et 0,49 g d'ester.

Alcool : $[\alpha]_D^{25}$ = +51° (c=0,63 ; $CHCl_3$). ee=98,1%.
Ester : ee=50%.

6.2. (*E*)-2-butènedioate (1:2) de (-)-6-chloro-3-[2-[(2,3-dihydro-1*H*-indén-2-yl)méthylamino]éthyl]-4-[3-(trifluorométhyl)benzoyl]-3,4-dihydro-2*H*-1,4-benzoxazine.

On opère dans des conditions analogues à celles décrites dans les exemples précédents, en traitant le (+)-6-chloro-4-[(3-trifluorométhyl)benzoyl]-3,4-dihydro-2*H*-1,4-benzoxazine-3-éthanol par le chlorure de thionyle, pour obtenir la 6-chloro-3-(2-chloroéthyl)-4-[(3-trifluorométhyl)benzoyl]-3,4-dihydro-2*H*-1,4-benzoxazine (dont on n'a pas déterminé le pouvoir rotatoire), puis en faisant réagir cette dernière avec la *N*-méthyl-2,3-dihydro-1*H*-indène-2-amine, puis en traitant la base avec de l'acide fumarique.

Point de fusion : 169-170°C $[\alpha]_D^{20}$ = -16,9° (c=1 ; $CH_3OH$).

Exemple 7 (Composé N°36)

(*E*)-2-butènedioate (1:2) de (±)-3-[2-[(2,3-dihydro-1*H*-indén-2-yl)amino]éthyl]-6-nitro-4-[3-(trifluorométhyl)benzoyl]-3,4-dihydro-2*H*-1,4-benzoxazine.

7.1. *N*-(2-hydroxy-5-nitrophényl)trifluoroacétamide.

On prépare une suspension de 18 g (0,116 mole) de 2-amino-4-nitrophénol dans 210 ml d'éther diéthylique, on ajoute 16 ml de pyridine, on refroidit le mélange par un bain de glace et d'éthanol et on ajoute, goutte à goutte, 24,5 g (0,116 mole) d'anhydride trifluoroacétique.
On laisse revenir le mélange à température ambiante, et on maintient l'agitation pendant une nuit.
On verse 200 ml d'eau glacée dans le mélange, on sépare la phase organique, on la lave successivement avec de l'acide chlorhydrique 1N, de l'eau, une solution saturée d'hydrogénocarbonate de sodium et une solution saturée de chlorure de sodium. Après séchage sur sulfate de magnésium on obtient 22,29 g de solide jaune qu'on utilise tel quel dans l'étape suivante.

7.2. (±)-6-Nitro-3,4-dihydro-2*H*-1,4-benzoxazine-3-acétate d'éthyle.

Dans un tricol de 1 l, sous agitation magnétique, refroidi à 0°C, on introduit 220 ml d'éthanol, on ajoute lentement, par petites portions, 3 g (0,133 mole) de sodium, puis on ajoute, goutte à goutte, successivement 22,29 g (0,088 mole) de *N*-(2-hydroxy-5-nitrophényl)trifluoroacétamide et 22,65 g (0,088 mole) de 4-bromobut-2-énoate d'éthyle pur à

75%, et on chauffe le mélange au reflux pendant 3h.

On évapore le solvant, on reprend le résidu avec 53 ml d'eau et 21 ml de soude 1N, et on l'extrait par de l'éther diéthylique. On lave la phase organique avec une solution saturée de chlorure de sodium, on la sèche sur sulfate de magnésium et on évapore le solvant. On obtient une huile rouge-orange qu'on purifie par chromatographie sur colonne de gel de silice en éluant avec un mélange de cyclohexane et d'acétate d'éthyle (85/15).

On obtient 9,25 g de produit.

7.3. (±)-6-Nitro-3,4-dihydro-2*H*-1,4-benzoxazine-3-éthanol.

Sous atmosphère d'argon on chauffe au reflux un mélange de 8,4 g (0,0317 mole) de (±)-6-nitro-3,4-dihydro-2*H*-1,4-benzoxazine-3-acétate d'éthyle, 3 g (0,762 mole) de borohydrure de sodium et 133 ml de 1,1-diméthyléthanol, on ajoute, goutte à goutte, 26,6 ml de méthanol et on poursuit le chauffage au reflux pendant 30 min.

On refroidit le mélange par un bain de glace, on ajoute 60 ml d'eau, on évapore le méthanol et le 1,1-diméthyléthanol, on extrait le mélange aqueux trois fois avec 120 ml d'acétate d'éthyle, on lave la phase organique deux fois avec 80 ml de solution saturée de chlorure de sodium, on la sèche sur sulfate de magnésium, on la filtre et on évapore le solvant sous pression réduite. on obtient 9,3 g de produit huileux qu'on purifie par chromatographie sur colonne de gel de silice en éluant avec un mélange de cyclohexane et d'acétate d'éthyle (60/40).

On obtient 5,0 g de produit qui cristallise au froid et qu'on utilise tel quel dans l'étape suivante.

7.4. (±)-6-Nitro-4-[3-(trifluorométhyl)benzoyl]-3,4-dihydro-2*H*-1,4-benzoxazine-3-éthanol.

Dans un ballon de 500 ml on introduit 40 ml de dichlorométhane, 5,16 g (0,023 mole) de (±)-6-nitro-3,4-dihydro-2*H*-1,4-benzoxazine-3-éthanol et 3,5 g (0,025 mole) de carbonate de potassium, on ajoute, goutte à goutte, 5,2 g (0,025 mole) de chlorure de 3-(trifluorométhyl)benzoyle en solution dans 40 ml de dichlorométhane, et on agite le mélange à température ambiante pendant 3h.

On ajoute 22 ml de soude 1N, on évapore le dichlorométhane et on extrait le résidu aqueux avec de l'acétate d'éthyle. Après lavage et séchage de la phase organique on évapore le solvant sous pression réduite et on purifie le résidu par chromatographie sur colonne de gel de silice en éluant avec un mélange de cyclohexane et d'acétate d'éthyle (7/3 à 5/5).

On obtient 5,4 g de produit pur.

7.5. (±)-3-(2-Chloroéthyl)-6-nitro-4-[3-(trifluorométhyl)benzoyl]-3,4-dihydro-2*H*-1,4-benzoxazine.

A 5,37 g (0,0135 mole) de (±)-6-nitro-4-[3-(trifluorométhyl)benzoyl]-3,4-dihydro-2*H*-1,4-benzoxazine-3-éthanol en solution dans 80 ml de dichlorométhane on ajoute, goutte à goutte, 4,85 ml (0,0675 mole) de chlorure de thionyle, et on agite le mélange en le tiédissant avec un bain d'eau chaude pendant 5h30. On ajoute encore 3 équivalents de chlorure de thionyle, on agite le mélange, on évapore le solvant, on reprend le résidu avec du toluène, on l'évapore, on lave le résidu à l'éther diisopropylique et on obtient 7 g de produit qu'on utilise tel quel dans l'étape suivante.

7.6. (*E*)-2-butènedioate (1:2) de (±)-3-[2-[(2,3-dihydro-1*H*-indén-2-yl)amino]éthyl]-6-nitro-4-[3-(trifluorométhyl)benzoyl]-3,4-dihydro-2*H*-1,4-benzoxazine.

A une solution de 1,58 g (0,0038 mole) de (±)-3-(2-chloroéthyl)-6-nitro-4-[3-(trifluorométhyl)benzoyl]-3,4-dihydro-2*H*-1,4-benzoxazine dans 15 ml de *N,N*-diméthylformamide, à température ambiante, sous atmosphère d'argon et sous agitation magnétique, on ajoute 0,69 g (0,0038 mole) de chlorhydrate de *N*-méthyl-2,3-dihydro-1*H*-indène-2-amine, 1,32 g (0,0095 mole) de carbonate de potassium et 0,63 g (0,0038 mole) d'iodure de potassium, et on chauffe le mélange à 110°C pendant 3h30.

On le refroidit, on ajoute 40 ml d'eau et 100 ml d'acétate d'éthyle, on sépare les phases, on extrait la phase aqueuse avec deux fois 50 ml d'acétate d'éthyle, on rassemble les phases organiques, on les lave deux fois avec 50 ml de solution saturée de chlorure de sodium, on les sèche sur sulfate de magnésium, on filtre et on évapore le solvant. On obtient 2,5 g de produit huileux qu'on purifie par chromatographie sur colonne de gel de silice en éluant avec un mélange de dichlorométhane et de méthanol (98/2). On obtient 1,36 g de base pure sous forme d'huile jaune. On prépare le fumarate en ajoutant un équivalent d'acide fumarique à la base en solution dans le propan-2-ol chaud, on l'isole et on le recristallise dans le méthanol.

On isole finalement 0,90 g de fumarate.

Point de fusion : 176-178°C.

Le tableau qui suit illustre les structures chimiques et les propriétés physiques de quelques composés selon l'invention. Dans la colonne "Sel", "-" désigne un composé à l'état de base, "ox." désigne un oxalate, ou éthanedioate, et "fum." désigne un fumarate, ou (*E*)-2-butènedioate ; le rapport indiqué entre parenthèses est le rapport molaire acide:base.

Tableau

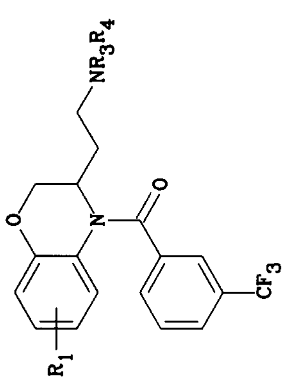

(I)

| N° | $R_1$ | $R_3$ | $R_4$ | Sel | F (°C) | Observations |
|---|---|---|---|---|---|---|
| 1 | H | 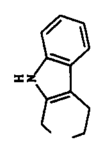 | | fum. (1:1) | 211-212 | - |
| 2 | 6-F | 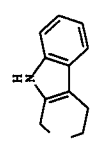 | | fum. (1:1) | 233-234 | - |
| 3 | 6-Cl | 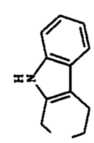 | | ox. (1,3:1) | 153-155 | - |
| 4 | 6-$CH_3$ | 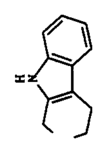 | | ox. (1,1:1) | 159-161 | - |
| 5 | 6-$OCH_3$ | 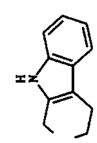 | | fum. (1:1) | 235-237 | - |
| 6 | H | 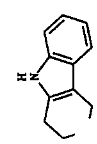 | | fum. (0,5:1) | 227-228 | - |

| N° | $R_1$ | $R_3$ | $R_4$ | Sel | F (°C) | Observations |
|---|---|---|---|---|---|---|
| 7 | 6-F | 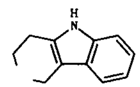 | | fum. (0,6:1) | 234-235 | - |
| 8 | 6-Cl | 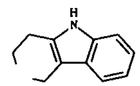 | | ox. (1:1) | 145-147 | - |
| 9 | 6-$CH_3$ | 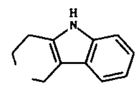 | | ox. (0,9:1) | 147-149 | - |
| 10 | 6-$OCH_3$ | 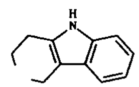 | | fum. (0,5:1) | 251-253 | - |
| 11 | H | 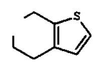 | | fum. (1,25:1) | 169-170 | - |
| 12 | 6-F | 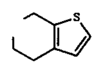 | | fum. (1:1) | 181-182 | - |
| 13 | H | 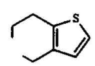 | | fum. (1:1) | 185-186 | - |
| 14 | 6-F | 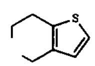 | | fum. (1:1) | 208-209 | - |
| 15 | 6-Cl | 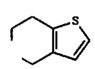 | | ox. (1:1) | 197-199 | - |

| N° | $R_1$ | $R_3$ | $R_4$ | Sel | F (°C) | Observations |
|---|---|---|---|---|---|---|
| 16 | 6-$CH_3$ | 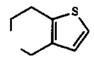 | | ox. (1:1) | 199-201 | - |
| 17 | H | 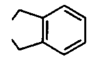 | | fum. (1:1) | 199-200 | - |
| 18 | H | $CH_3$ | 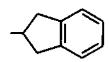 | ox. (1,5:1) | 154-155 | - |
| 19 | 6-F | $CH_3$ | 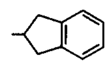 | ox. (1:1) | 153-154 | - |
| 20 | 6-Cl | $CH_3$ | 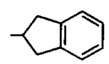 | ox. (0,9:1) | 173-175 | - |
| 21 | 6-Cl | $CH_2CH_2CH_3$ | 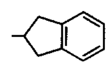 | ox. (1:1) | 127-129 | - |
| 22 | 6-$CH_3$ | $CH_3$ | 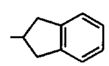 | ox. (1:1) | 158-160 | - |
| 23 | 6-$CH_3$ | $CH_2CH_2CH_3$ | 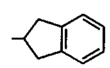 | ox. (1:1) | 89-91 | - |
| 24 | 6-$OCH_3$ | $CH_3$ | 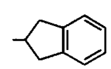 | ox. (1:1) | 185-187 | - |

| N° | $R_1$ | $R_3$ | $R_4$ | Sel | F (°C) | Observations |
|---|---|---|---|---|---|---|
| 25 | 6-$OCH_3$ | $CH_2CH_2CH_3$ | 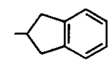 | ox. (1:1) | 139-141 | - |
| 26 | H | H | 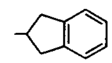 | ox. (1:1) | 193-194 | - |
| 27 | 6-Cl | H | 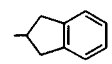 | fum. (1:1) | 206-207 | - |
| 28 | H | $CH_3$ | 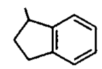 | ox. (1:1) | 152-153 | 1 seul diastéréo-isomère |
| 29 | 6-Cl | $CH_3$ | 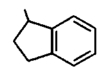 | fum. (1:1) | 188-189 | mélange 90:10 de diastéréoisomères |
| 30 | 6-Cl | $CH_3$ | 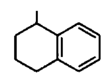 | fum. (1:1) | 145-146 | mélange 65:35 de diastéréoisomères |
| 31 | H | $CH_3$ | 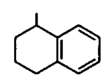 | ox. (1:1) | 142-143 | mélange 54:46 de diastéréoisomères |
| 32 | 7-$CH_3$ | $CH_3$ | 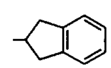 | fum. (0,5:1) | 163-165 | - |
| 33 | 7-$CH_3$ | $CH_3$ | 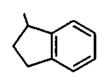 | fum. (1:1) | 165-167 | mélange 60:40 de diastéréoisomères |

| N° | $R_1$ | $R_3$ | $R_4$ | Sel | F (°C) | Observations |
|---|---|---|---|---|---|---|
| 34 | 7-$CH_3$ | H | 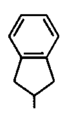 | ox. (1,1:1) | 208-210 | - |
| 35 | 6-$NO_2$ | $CH_3$ | 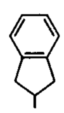 | ox. (1,1:1) | 194-196 | - |
| 36 | 6-$NO_2$ | H | 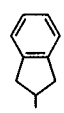 | fum. (0,5:1) | 176-178 | - |
| 37 | 6-$NO_2$ | $CH_3$ | 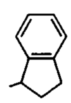 | fum. (1:1)<br>- | 178-180<br>138-140 | diastéréoisomère A<br>diastéréoisomère B |
| 38 | 6-Cl | $CH_3$ | 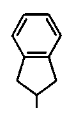 | fum. (0,5:1) | 168-169<br>$[\alpha]_D^{20}$ = +16,2°<br>(c=1 ; $CH_3OH$) | énantiomère dextrogyre |
| 39 | 6-Cl | $CH_3$ | 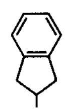 | fum. (0,5:1) | 169-170<br>$[\alpha]_D^{20}$ = -16,9°<br>(c=1 ; $CH_3OH$) | énantiomère lévogyre |

Les composés de l'invention ont fait l'objet de divers essais qui ont mis en évidence leur intérêt comme substances thérapeutiques.

Ainsi ils ont été soumis au test de l'ischémie cérébrale globale chez la souris. L'ischémie est due à un arrêt

cardiaque induit par une injection intraveineuse rapide de chlorure de magnésium. Dans ce test on mesure le "temps de survie", c'est-à-dire l'intervalle entre le moment de l'injection de chlorure de magnésium et le dernier mouvement respiratoire observable de chaque souris. Ce dernier mouvement est considéré comme l'indice ultime d'une fonction du système nerveux central.

L'arrêt respiratoire apparaît approximativement 19 secondes après l'injection de chlorure de magnésium.

Des souris mâles (Swiss OF1 IFFA CREDO) sont étudiées par groupes de 10. Elles sont nourries et abreuvées ad libitum avant les essais. Le temps de survie est mesuré 10 minutes après l'administration intrapéritonéale des composés de l'invention. Les résultats sont donnés sous la forme de la différence entre le temps de survie mesuré dans un groupe de 10 souris ayant reçu le composé et le temps de survie mesuré dans un groupe de 10 souris ayant reçu le liquide véhicule. Les rapports entre les modifications dans le terme de survie et la dose du composé sont enregistrés graphiquement selon une courbe semilogarithmique.

Cette courbe permet le calcul de la "dose efficace 3 secondes" ($DE_{3''}$), c'est-à-dire la dose (en mg/kg) qui produit une augmentation de 3 secondes du temps de survie par rapport au groupe témoin de 10 souris non traitées.

Une augmentation de 3 secondes du temps de survie est à la fois significative statistiquement et reproductible.

Les $DE_{3''}$ des composés de l'invention vont de 0,1 à 30 mg/kg par voie intrapéritonéale.

Les composés de l'invention ont également fait l'objet d'une étude des courants du baryum potentiel-dépendants ("voltage-dépendent") par la technique dite du "patch-clamp".

La mesure des courants du baryum passant par les canaux calciques potentiel-dépendants est effectuée sur une préparation de cellules de cortex de rat nouveau-né (Sprague-Dawley) en culture (6 à 10 jours de culture) ; il s'agit, dans le cas de ces cellules, de courants composites mettant en jeu les canaux L, N et P, comme décrit dans *Soc. Neurosci. Abstr.* (1989) **15** 823.

Les chambres de mesure, d'un volume de 800 µl, contenant les cellules de cortex, sont placées sur la platine d'un microscope inversé Olympus IMT-2™ et les cellules sont observées au grossissement 400×. Les chambres sont continuellement perfusées (4 à 5 ml/min) à l'aide d'un dispositif distributeur de solutions acceptant 9 entrées (volume mort<50 µl) dont la sortie unique, constituée d'un tube de polyéthylène de 500 µm d'ouverture, est placée à moins de 3 mm de la cellule étudiée. Ce dispositif a l'avantage de permettre un échange rapide de solution au niveau des cellules étudiées.

La méthode de patch-clamp utilisée est décrite dans *Pfluegers Archives* (1981) **391** 85-100. Un amplificateur Axopatch-1D™ associé à un ordinateur de type AT 386-33MHz, utilisant les logiciels PCLAMP™ de Axon Instruments™ est employé pour la stimulation des cellules, l'acquisition des données et l'analyse des résultats. Pour la réalisation des enregistrements des courants du baryum, des pipettes en verre borosilicaté sont approchées des cellules grâce à un micromanipulateur hydraulique Narishige WR 60™. La pointe des pipettes est remplie de la solution intracellulaire de référence dont la composition (en mM) est la suivante : CsCl (140), $CaCl_2$ (1), $Na_2ATP$ (4), EGTA (11 ; pCa=8), Hepes (10), Tris-OH (pH=7,2).

Une fois que la configuration dite "cellule" entière (whole cell) est obtenue, la cellule est perfusée avec une solution dite TEA-Baryum dont la composition (en mM) est la suivante : TEA-Cl (144), $BaCl_2$ (5), $MgCl_2$ (2), CsCl (3), glucose (10), Hepes (10), Tris-OH (pH=7,4).

Cette solution permet la mesure du courant calcique (assimilé au courant du baryum passant par les canaux calciques potentiel-dépendants) tout en s'affranchissant des courants sodiques et potassiques.

Le courant du baryum potentiel-dépendant global est obtenu par l'application d'un saut dépolarisant de potentiel d'une durée de 250 ms, portant le potentiel de membrane de -80 mV à 0 mV. La fréquence de stimulation est de 0,25 Hz.

Les composés de l'invention sont mis en solution dans le milieu TEA-baryum et appliqués une fois l'amplitude du courant du baryum stabilisée. Après obtention d'un effet inhibiteur stable, la cellule est de nouveau perfusée avec la solution TEA-baryum de contrôle afin d'observer la réversion de l'effet.

La puissance de l'effet obtenu est comparée à celle d'une solution de cadmium à 100 µM. L'inhibition du courant baryum potentiel-dépendant varie en fonction des doses de composés étudiés et, pour les composés les plus actifs, est de l'ordre de 40% à la concentration de 1 µM et de 90% à la concentration de 10 µM.

Les résultats des essais effectués sur les composés de l'invention montrent que, *in vitro*, ils ont des propriétés antagonistes calciques neuronales et, *in vivo*, ils ont des propriétés neuroprotectrices et anti-ischémiques.

Ils suggèrent que les composés peuvent être utilisés pour le traitement et la prévention de désordres cérébraux tels que ceux qui sont consécutifs, par exemple, à une attaque ischémique, un arrêt cardiaque ou respiratoire, une thrombose ou une embolie cérébrale, pour le traitement de la sénilité cérébrale, de la démence consécutive aux infarctus multiples, de la démence sénile, par exemple de la maladie d'Alzheimer ou de la maladie de Pick, pour le traitement de l'atrophie olivo-ponto-cérébellaire et d'autres maladies neurodégénératives telles que la chorée de Huntington, la sclérose latérale amyotrophique, pour le traitement des traumatismes crâniens ou spinaux, pour la prévention des dommages neuronaux faisant suite à des états convulsifs, pour le traitement de certains cancers, pour le traitement des altérations neurologiques dues au SIDA, et pour la prévention et le traitement des rétinopathies diabétiques, de la

dégénérescence du nerf optique et des rétinopathies associées au glaucome, et en général pour le traitement de toute pathologie liée à un dysfonctionnement de l'homéostase calcique neuronale.

A cet effet ils peuvent être présentés sous toutes formes pharmaceutiques adaptées à l'administration entérale ou parentérale, en association avec des excipients appropriés, par exemple sous forme de comprimés, dragées, gélules, capsules, suppositoires, solutions ou suspensions buvables ou injectables, dosés pour permettre une administration journalière de 1 à 1000 mg de substance active.

**Revendications**

1. Composé, sous forme d'isomère optique pur ou de mélange d'isomères optiques, répondant à la formule générale (I)

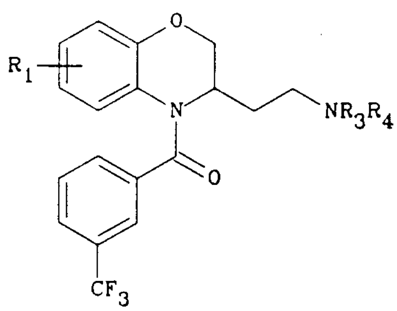

(I)

dans laquelle

$R_1$ représente un atome d'hydrogène, de fluor ou de chlore ou un groupe méthyle, alcoxy en $C_1$-$C_3$ ou nitro,
$R_3$, pris isolément, représente un atome d'hydrogène ou un groupe alkyle en $C_1$-$C_3$,
$R_4$, pris isolément, représente un groupe 2,3-dihydro-1*H*-indén-2-yle, un groupe 2,3-dihydro-1*H*-indén-1-yle ou un groupe 1,2,3,4-tétrahydronaphtalén-1-yle,
ou bien
$R_3$ et $R_4$ forment ensemble, et avec l'atome d'azote qui les porte, un groupe 1,2,3,4-tétrahydro-9*H*-pyrido[3,4-*b*]indol-2-yle, un groupe 1,2,3,4-tétrahydro-9*H*-pyrido[4,3-*b*]indol-3-yle, un groupe 4,5,6,7-tétrahydrothiéno[2,3-*c*]pyridin-6-yle, un groupe 4,5,6,7-tétrahydrothiéno[3,2-*c*]pyridin-6-yle ou un groupe 2,3-dihydro-1*H*-isoindol-2-yle, dont les formules respectives sont les suivantes :

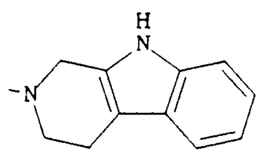
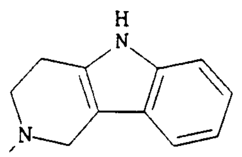
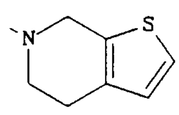
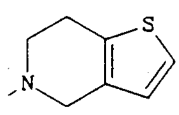
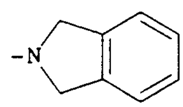

à l'état de base ou de sel d'addition à un acide.

2. Composé selon la revendication 1, caractérisé en ce que $R_1$ représente un atome de fluor ou de chlore ou un groupe méthyle ou méthoxy, $R_3$ représente un atome d'hydrogène ou un groupe alkyle en $C_1$-$C_3$ et $R_4$ représente un groupe 2,3-dihydro-1*H*-indén-2-yle, un groupe 2,3-dihydro-1*H*-indén-1-yle ou un groupe 1,2,3,4-tétrahydronaphtalén-1-yle.

3. Procédé de préparation de composés selon la revendication 1, caractérisé en ce qu'on fait réagir un 2-aminophénol de formule générale (II)

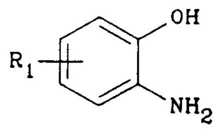

(II)

dans laquelle $R_1$ est tel que défini dans la revendication 1, avec l'anhydride trifluoracétique, pour obtenir un amide de formule générale (IV)

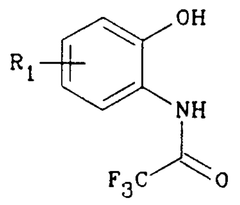

(IV)

que l'on fait réagir avec le 4-bromobut-2-énoate d'éthyle, puis on réduit la fonction ester du dérivé de 3,4-dihydro-2*H*-1,4-benzoxazine-3-acétate d'éthyle ainsi obtenu, de formule générale (VI)

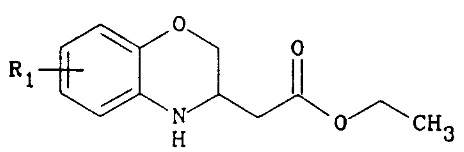

(VI)

avec un agent réducteur, pour obtenir le dérivé de 3,4-dihydro-2*H*-1,4-benzoxazine-3-éthanol de formule générale (VII)

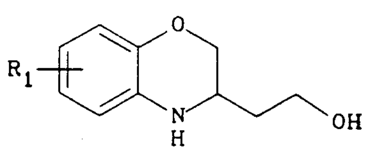

(VII)

que l'on fait réagir avec le chlorure d'acide 3-(trifluorométhyl)benzoïque pour obtenir un alcool de formule générale (IX)

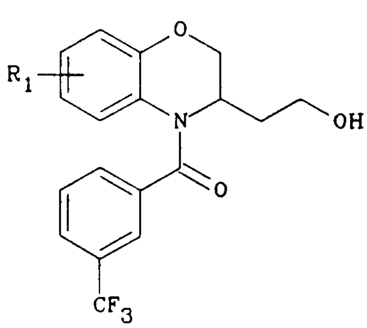

(IX)

que l'on fait réagir avec du chlorure de thionyle pour obtenir un composé de formule générale (X)

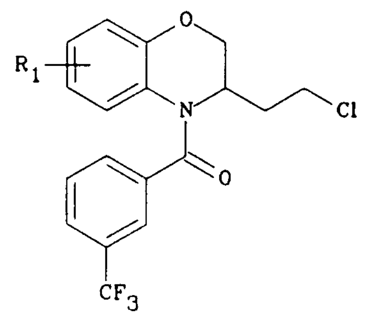

(X)

et finalement, on fait réagir ce dernier avec une amine de formule générale $HNR_3R_4$, dans laquelle $R_3$ et $R_4$ sont tels que définis dans la revendication 1.

**4.** Médicament caractérisé en ce qu'il consiste en un composé selon la revendication 1.

**5.** Composition pharmaceutique caractérisée en ce qu'elle contient un composé selon la revendication 1, associé à un excipient.

**Claims**

**1.** Compound, either in the form of a pure optical isomer or a mixture of optical isomers, of the general formula (I)

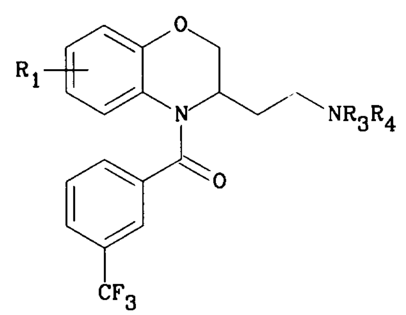

(I)

wherein

$R_1$ represents a hydrogen, fluorine or chlorine atom or a methyl, $C_1$-$C_3$ alkoxy or nitro group,
$R_3$ alone represents a hydrogen atom or a $C_1$-$C_3$ alkyl group,
$R_4$ alone represents a 2,3-dihydro-1*H*-inden-2-yl group, a 2,3-dihydro-1*H*-inden-1-yl group or a 1,2,3,4-tetrahydronaphthalen-1-yl group,
or alternatively
$R_3$ and $R_4$ together complete, with the nitrogen atom to which they are attached, a 1,2,3,4-tetrahydro-9*H*-pyrido[3,4-*b*]indol-2-yl group, a 1,2,3,4-tetrahydro-9*H*-pyrido[4,3-*b*]indol-3-yl group, a 4,5,6,7-tetrahydrothieno[2,3-*c*]pyridin-6-yl group, a 4,5,6,7-tetrahydrothieno[3,2-*c*]pyridin-6-yl group or a 2,3-dihydro-1*H*-isoindol-2-yl group, the respective formulae of which are as follows:

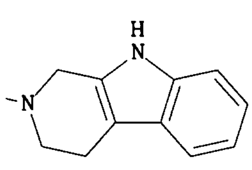 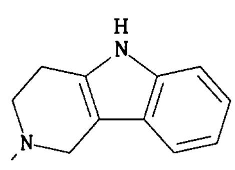 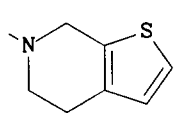 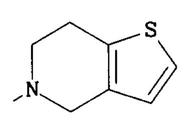 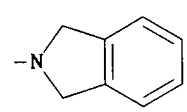

in the form of a base or an addition salt with an acid.

2. Compound according to claim 1, wherein $R_1$ represents a fluorine or chlorine atom or a methyl or methoxy group, $R_3$ represents a hydrogen atom or a $C_1$-$C_3$ alkyl group and $R_4$ represents a 2,3-dihydro-1*H*-inden-2-yl group, a 2,3-dihydro-1*H*-inden-1-yl group or a 1,2,3,4-tetrahydronaphthalen-1-yl group.

3. process for the preparation of a coumpound according to claim 1 wherein a 2-aminophenol of general formula (II)

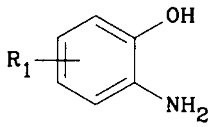

(II)

wherein $R_1$ is as defined in claim 1, is reacted with trifluoroacetic anhydride, in order to obtain an amide of general formula (IV)

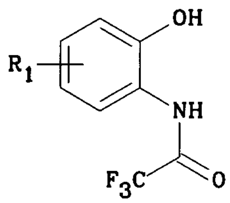

(IV)

which is reacted with ethyl 4-bromo-2-butenoate, followed by reduction of the ester function of the ethyl 3,4-dihydro-2*H*-1,4-benzoxazine-3-acetate derivative thus obtained, of general formula (VI)

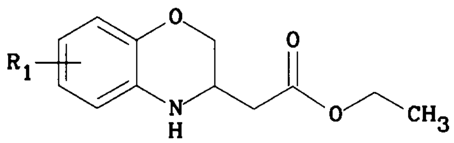

(VI)

with a reducing agent, in order to obtain the 3,4-dihydro-2*H*-1,4-benzoxazine-3-ethanol derivative of general formula (VII)

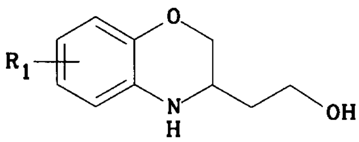

(VII)

which is reacted with 3-(trifluoromethyl)benzoyl chloride in order to obtain an alcool of general formula (IX)

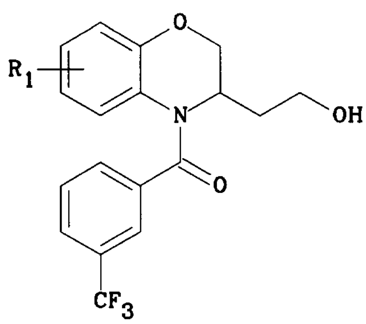

(IX)

which is reacted with thionyl chloride in order to obtain a compound of general formula (X)

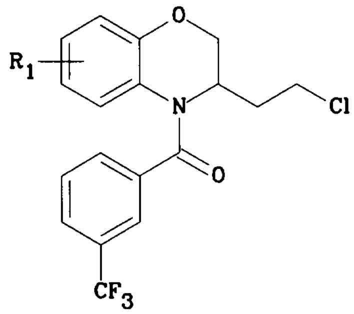

(X)

and, finally, this compound is reacted with an amine of general formula $HNR_3R_4$, wherein $R_3$ and $R_4$ are as defined in claim 1.

4. Medicament which consists of a compound according to claim 1.

5. Pharmaceutical composition which contains a compound according to claim 1 in combination with an excipient.

**Patentansprüche**

1. Verbindungen, in Form des reinen optischen Isomeren oder einer Mischung von optischen Isomeren, der allgemeinen Formel (I)

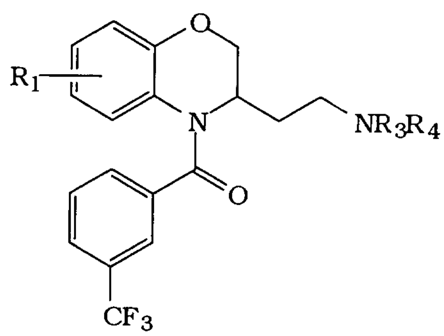

(I)

in der

$R_1$ ein Wasserstoff-, Fluor- oder Chloratom, eine Methylgruppe, eine $C_1$-$C_3$-Alkoxygruppe oder eine Nitrogruppe,
$R_3$ allein ein Wasserstoffatom oder eine $C_1$-$C_3$-Alkylgruppe,
$R_4$ allein eine 2,3-Dihydro-1*H*-inden-2-yl-gruppe, eine 2,3-Dihydro-1*H*-inden-1-yl-gruppe oder eine 1,2,3,4-Tetrahydronaphthalin-1-yl-gruppe
oder
$R_3$ und $R_4$ gemeinsam mit dem sie tragenden Stickstoffatom eine 1,2,3,4-Tetrahydro-9*H*-pyrido[3,4-*b*]indol-2-yl-, 1,2,3,4-Tetrahydro-9*H*-pyrido[4,3-*b*]indol-3-yl-, 4,5,6,7-Tetrahydrothieno[2,3-*c*]pyridin-6-yl-, 4,5,6,7-Tetrahydrothieno[3,2-*c*]pyridin-6-yl- oder 2,3-Dihydro-1*H*-isoindol-2-yl-gruppe, deren entsprechende Formeln die folgenden sind:

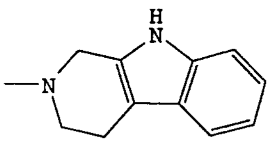

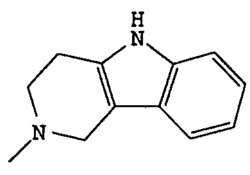

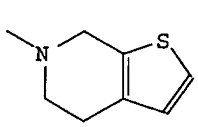

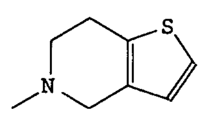

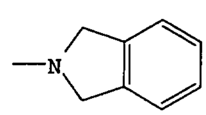

bedeuten, in Form der Base oder des Additionssalzes mit einer Säure.

**2.** Verbindung nach Anspruch 1, dadurch gekennzeichnet, daß $R_1$ ein Fluoratom oder ein Chloratom oder eine Methylgruppe oder eine Methoxygruppe, $R_3$ ein Wasserstoffatom oder eine $C_1$-$C_3$-Alkylgruppe und $R_4$ eine 2,3-Dihydro-1*H*-inden-2-yl-, 2,3-Dihydro-1*H*-inden-1-yl- oder 1,2,3,4-Tetrahydronaphthalin-1-yl-gruppe bedeuten.

**3.** Verfahren zur Herstellung der Verbindungen nach Anspruch 1, dadurch gekennzeichnet, daß man ein 2-Aminophenol der allgemeinen Formel (II)

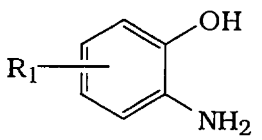

(II)

in der $R_1$ die in Anspruch 1 angegebenen Bedeutungen besitzt, mit Trifluoressigsäureanhydrid umsetzt zur Bildung eines Amids der allgemeinen Formel (IV)

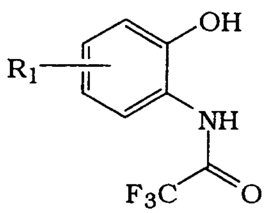

(IV)

welches man mit 4-Brombut-2-ensäureethylester umsetzt und dann die Esterfunktion des in dieser Weise erhaltenen 3,4-Dihydro-2*H*-1,4-benzoxazin-3-essigsäure-ethylester-Derivats der allgemeinen Formel (VI)

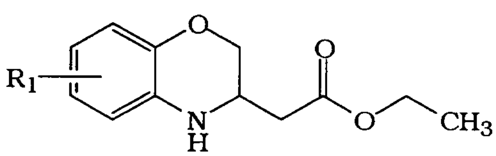

(VI)

mit einem Reduktionsmittel reduziert zur Bildung des 3,4-Dihydro-2*H*-1,4-benzoxazin-3-ethanol-Derivats der allgemeinen Formel (VII)

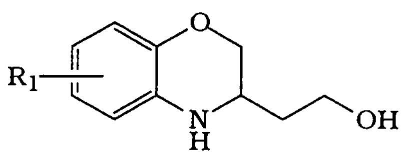

(VII)

welches man mit 3-(Trifluormethyl)-benzoesäurechlorid umsetzt zur Bildung eines Alkohols der allgemeinen Formel (IX)

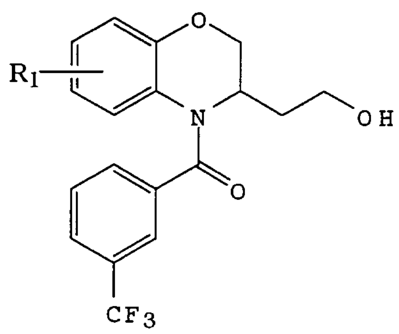

(IX)

welchen man mit Thionylchlorid umsetzt zur Bildung einer Verbindung der allgemeinen Formel (X)

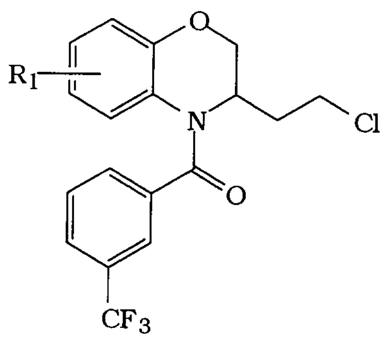

(X)

welch letztere Verbindung man schließlich mit einem Amin der allgemeinen Formel $HNR_3R_4$, worin $R_3$ und $R_4$ die in Anspruch 1 angegebenen Bedeutungen besitzen, umsetzt.

4. Arzneimittel, dadurch gekennzeichnet, daß es aus einer Verbindung nach Anspruch 1 besteht.

5. Pharmazeutische Zubereitung, dadurch gekennzeichnet, daß sie eine Verbindung nach Anspruch 1 in Kombination mit einem Trägermaterial enthält.